# EUROPEAN PATENT APPLICATION

(11) **EP 4 254 424 A1**
(43) Date of publication of application: **04.10.2023**
(21) Application number: 23163437.9
(22) Date of filing: 22.03.2023
(51) Int. Cl.: G16H 50/20, G06F 16/901, G06N 20/00, G16H 50/30, G16H 50/70

(54) **INFORMATION PROCESSING APPARATUS, INFORMATION PROCESSING METHOD, AND PROGRAM THEREOF**

(30) Priority: 29.03.2022 JP 2022053171
(71) Applicant: Rakuten Group, Inc., Setagaya-ku Tokyo 158-0094 (JP)
(72) Inventor: Hirate, Yu, Setagaya-ku, Tokyo, 158-0094 (JP); Rahman, Md Mostafizur, Setagaya-ku, Tokyo, 158-0094 (JP); Ebisu, Takuma, Setagaya-ku, Tokyo, 158-0094 (JP); Kondapaka, Manoj, Setagaya-ku, Tokyo, 158-0094 (JP); Kikuta, Daisuke, Setagaya-ku, Tokyo, 158-0094 (JP); Abrol, Satyen, Setagaya-ku, Tokyo, 158-0094 (JP)
(74) Representative: Hoffmann Eitle

(57) **Abstract**

An information processing apparatus acquires factual features of each of a plurality of users as user features, creates a relationship graph indicating a social relationship among the plurality of users based on the user features, sets a target user among the plurality of users, and predicts a disease risk indicating the risk of developing at least one predetermined disease for the target user, based on the relationship graph and the user feature of the target user.

## Description

### TECHNICAL FIELD

The present invention relates to an information processing apparatus, an information processing method, and a program thereof, and particularly to a technique for predicting disease risk.

### BACKGROUND ART

In recent years, a technique has been developed in which a network such as the Internet is used to provide a service relating to a predetermined disease for a user at risk of developing the disease. For example, JP 2019-153222A discloses a technique of acquiring behavior information indicating behavior on a network of a user (risk user) at risk for developing a predetermined disease, and providing information relating to the predetermined disease to another user (target user) having behavior information related to the behavior information.

JP 2019-153222Ais an example of related art.

### SUMMARY OF THE INVENTION

According to JP 2019-153222A, based on information on the behavior of the risk user and the target user, it is estimated that the target user has the same risk of developing the disease as the risk user, and the target user is provided with information relating to the disease. However, the information on the behavior is merely local information between the risk user and the target user, and thus there is room for improvement in predicting the risk of developing a disease based on more multifaceted information.

The present invention has been made in view of the above problems, and aims to provide a technique for assisting, in a more multifaceted manner, prediction of a user's risk of developing a disease based on comprehensive relationships between the user and a plurality of other users.

In order to solve the above problem, one aspect of an information processing apparatus according to the present invention includes: acquisition means for acquiring factual features of each of a plurality of users as user features; creation means for creating a relationship graph indicating a social relationship between the plurality of users based on the user features; target user setting means for setting a target user among the plurality of users; and prediction means for, based on the relationship graph and the user feature of the target user, predicting a disease risk indicating a risk of developing at least one disease for the target user.

The prediction means may predict the disease risk for the target user by using a machine learning model configured to receive the user feature of the target user as input and output the disease risk for the target user.
training means may be further included, which is for training the machine learning model, and the training means may train the machine learning model using a disease feature obtained from the relationship graph.

The disease feature obtained from the relationship graph may include information indicating whether or not each of the plurality of users has contracted the at least one predetermined disease.

In the relationship graph, each user may be indicated by a user node, and the creation means may connect nodes with links based on the factual features.

The creation means may connect a pair of user nodes having the same factual feature with an explicit link, and connect, with an implicit link, a pair of nodes that are not linked with the explicit link, based on a plurality of pairs of user nodes that are connected with the explicit links.

The creation means may determine a closeness of the connected pair based on at least one factual feature shared by the pair.

The disease risk may be represented by a numeric value from 0 to 1 for each of the at least one disease, where 1 is a maximum likelihood.

In order to solve the above problem, one aspect of an information processing method according to the present invention includes: acquiring factual features of each of a plurality of users as user features; creating a relationship graph indicating a social relationship between the plurality of users based on the user features; setting a target user among the plurality of users; and predicting, based on the relationship graph and the user feature of the target user, a disease risk indicating a risk of developing at least one disease for the target user.

In order to solve the above problem, one aspect of a program according to the present invention is an information processing program for causing a computer to execute information processing, the program causing the computer to execute: acquisition processing for acquiring factual features of each of a plurality of users as user features; creation processing for creating a relationship graph indicating a social relationship between the plurality of users based on the user features; target user setting processing for setting a target user among the plurality of users; and prediction processing for predicting, based on the relationship graph and the user feature of the target user, a disease risk indicating a risk of developing at least one disease for the target user.

According to this invention, it is possible to, in a more multifaceted manner, assist prediction of a user's risk of developing a disease.

The objects, aspects, and effects of the present invention described above and objects, aspects and effects of the present invention not described above can be understood by a person skilled in the art based on the following modes for carrying out the invention by referring to the accompanying drawings and the description of the claims.

### BRIEF DESCRIPTION OF THE DRAWINGS

FIG. 1 shows a configuration example of an information processing system.
FIG. 2 shows an example of a functional configuration of an information processing apparatus 10 according to an embodiment.
FIG. 3 shows a flowchart of processing for creating a relationship graph.
FIG. 4A is a diagram for illustrating an explicit link.
FIG. 4B is a diagram for illustrating an explicit link.
FIG. 4C is a diagram for illustrating an explicit link.
FIG. 4D is a diagram for illustrating an implicit link.
FIG. 5A is a diagram for illustrating processing for inferring a relationship between links.
FIG. 5B shows a flowchart of an example of processing for grouping pairs into clusters.
FIG. 6A shows a conceptual diagram of a score (closeness score) based on closeness of a relationship for a user pair.
FIG. 6B shows schematic architecture of a score prediction model 112.
FIG. 7 shows a conceptual diagram of a relationship graph.
FIG. 8A is a diagram for illustrating a training stage of a disease risk prediction model 111.
FIG. 8B is a diagram for illustrating a disease risk prediction stage for a target user.
FIG. 9 shows a hardware configuration example of an information processing apparatus 10 and a user apparatus 11.
FIG. 10 shows a flowchart of processing executed by the information processing apparatus 10.

### EMBODIMENTS OF THE INVENTION

Hereinafter, an embodiment for implementing the present invention will be described in detail with reference to the accompanying drawings. Constituent elements disclosed hereinafter that have the same function as each other are denoted by identical reference signs, and description thereof is omitted. Note that the embodiment disclosed hereinafter is an example serving as a means of realizing the present invention, the embodiment is to be amended or modified as appropriate according to the configuration of the apparatus to which the present invention is applied and various conditions, and the present invention is not limited to the following embodiment. Also, not all combinations of features described in the present embodiment are essential for the solving means of the present invention.

### Functional Configuration of Information Processing Apparatus

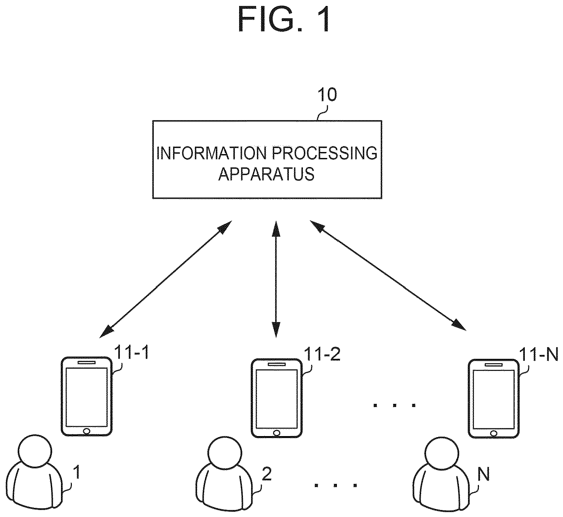

FIG. 1 shows an example of a configuration of an information processing system according to the present embodiment. In one example, as shown in FIG. 1, the present information processing system includes an information processing apparatus 10, and a plurality of user apparatuses 11-1 to 11-N (N>1) used by any plurality of users 1 to N. Note that in the following description, the user apparatuses 11-1 to 11-N can be referred to collectively as user apparatuses 11 unless otherwise specified. Also, in the following description, the terms "user apparatus" and "user" can be used synonymously.

The user apparatus 11 is, for example, an apparatus such as a smartphone or a tablet, and can communicate with the information processing apparatus 10 via a public network such as LTE (Long Term Evolution) or a wireless communication network such as a wireless LAN (Local Area Network). The user apparatus 11 has a display unit (display screen) such as a liquid crystal display, and each user can perform various operations through a GUI (Graphic User Interface) installed in the liquid crystal display. The operations include various operations performed with a finger or a stylus on content such as images displayed on the screen, such as a tap operation, a slide operation, or a scroll operation.

Note that the user apparatus 11 is not limited to an apparatus of the form shown in FIG. 1, and may also be an apparatus such as a desktop PC (Personal Computer) or a laptop PC. In this case, the operations performed by each user can be performed using an input apparatus such as a mouse or a keyboard. Also, the user apparatus 11 may include a display screen separately.

The user apparatus 11 can use a service by logging into a web service (Internet-related service) provided via the information processing apparatus 10, from the information processing apparatus 10 or another apparatus (not shown). The web service can include an online mall, an online supermarket, or a service relating to communication, finance, real estate, sports, or travel, which are provided via the Internet. The user apparatus 11 can transmit information relating to the user of the user apparatus 11 to the information processing apparatus 10 by using such a web service.

For example, the user apparatus 11 can transmit feature information relating to the user apparatus or the user, such as the IP (Internet Protocol) address of the user apparatus 11, the address of the user, or the name of the user, to the information processing apparatus 10.

Also, the user apparatus 11 can perform positioning calculation based on signals or the like received from GPS (Global Positioning System) satellites (not shown), generate information obtained through the calculation as position information of the user apparatus 11, and transmit the generated information to the information processing apparatus 10.

The information processing apparatus 10 acquires various types of information from the user apparatus 11, and based on the information, creates a relationship graph network (hereinafter referred to as a relationship graph) showing social relationships between users. Then, the information processing apparatus 10 predicts the risk of developing any disease (hereinafter referred to as disease risk) for any user using the created relationship graph.

### Functional Configuration of Information Processing Apparatus 10

The information processing apparatus 10 according to the present embodiment first acquires various types of information from the user apparatuses 11-1 to 11-N and creates a relationship graph showing social relationships between the users 1 to N. Then, the information processing apparatus 10 identifies a target user among the users 1 to N. The information processing apparatus 10 predicts the disease risk of the target user by applying the features of the created social graph to a trained machine learning model.

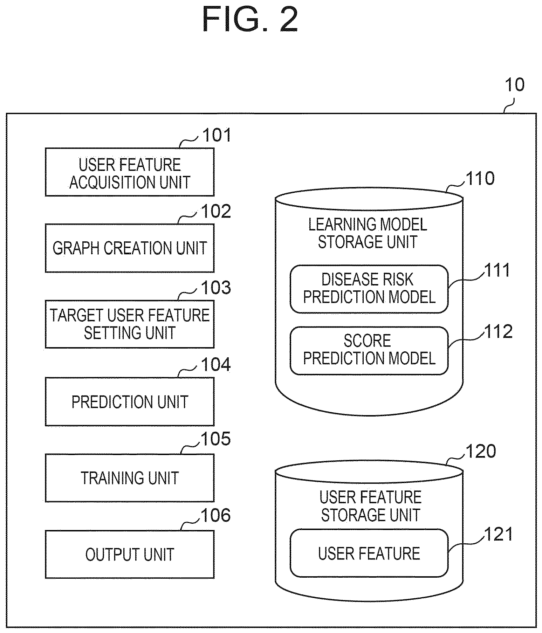

FIG. 2 shows an example of a functional configuration of the information processing apparatus 10 according to this embodiment.

The information processing apparatus 10 shown in FIG. 2 includes a user feature acquisition unit 101, a graph creation unit 102, a target user feature setting unit 103, a prediction unit 104, a training unit 105, an output unit 106, a learning model storage unit 110, and a user feature storage unit 120. The learning model storage unit 110 stores a disease risk prediction model 111 and a score prediction model 112. The various learning models will be described later. Also, the user feature storage unit 120 stores user features 121.

The user feature acquisition unit 101 acquires factual features (factual information) (hereinafter referred to as user features) about the user apparatuses or the users from each of the user apparatuses 11-1 to 11-N. The user features are features (information) based on facts actually or objectively acquired from the user apparatuses or the users. For example, the user feature acquisition unit 101 can directly acquire the user features from the user apparatuses 11. Also, the user feature acquisition unit 101 can acquire the user features as information registered with a predetermined web service by the users of the user apparatuses 11.

The user features include IP addresses of the user apparatuses, the addresses of the users or the names of the users, the numbers of credit cards possessed by the users, demographic information of the users (demographic user attributes such as sex, age, residential area, occupation, and family composition), and the like. The user features also include features relating to user health, such as physical examination data and daily health data. Such health-related features include, for example, height and weight, sleep time, dietary information (caloric intake, etc.), blood type, blood pressure, and medical history, and for example, can be acquired with use of data registered in a health-related service, which is one web service.

Also, the user features may include registration numbers and registration names used when using a predetermined web service. Also, the user features may include information relating to a call history, a delivery address other than the address of the user for a product at the time of using the predetermined web service, a use status during use of the predetermined web service, a use history, a search history, and points that can be accumulated through use of a service. Thus, the user features can include any information, including information relating to the user apparatus or the user, and information relating to use of a predetermined service through communication.

The user feature acquisition unit 101 stores the acquired user features in the user feature storage unit 120 as the user features 121.

The graph creation unit 102 uses the various user features acquired by the user feature acquisition unit 101 to create a relationship graph. The relationship graph will be described later.

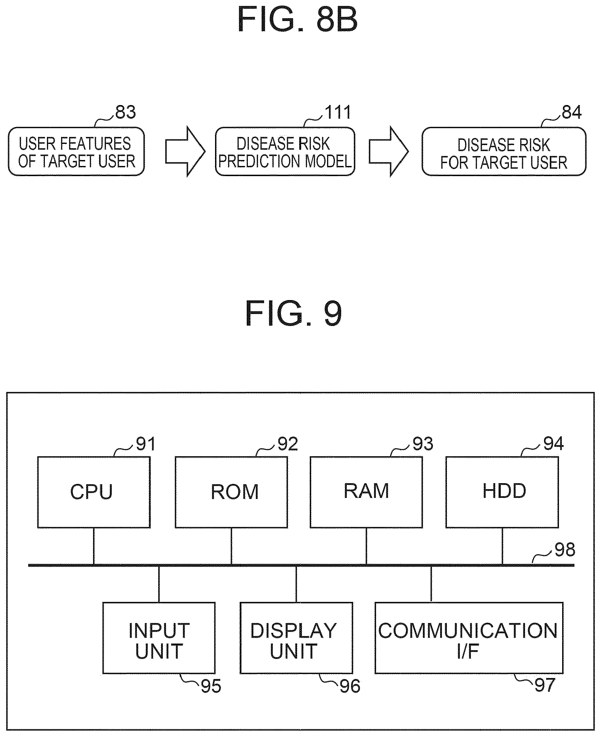

The target user feature setting unit 103 performs setting of a user (hereinafter referred to as a target user) whose disease risk is to be predicted. The target user may be set by an operator through an input operation performed using an input unit (input unit 95 in FIG. 9), may be set in advance in the system, or may be set by any program stored in a storage unit (ROM 92 or RAM 93 in FIG. 9). Furthermore, the target user feature setting unit 103 acquires the user feature of the target user from the user features 121 and sets the acquired user feature in the prediction unit 104.

The prediction unit 104 predicts the disease risk indicating the risk of developing (contracting) at least one predetermined disease for the target user set by the target user feature setting unit 103. In this embodiment, the disease risk prediction model 111 that has been trained by the training unit 105 is used to predict the disease risk of the target user. The disease risk prediction processing will be described later.

The training unit 105 trains the disease risk prediction model 111 and the score prediction model 112 and stores the trained disease risk prediction model 111 and score prediction model 112 in the learning model storage unit 110. The training processing of each learning model will be described later.

The output unit 106 outputs the disease risk prediction result for the target user predicted by the prediction unit 104. The output unit 106 may generate and output information about the disease risk. The output may be any output processing, may be output to an external apparatus via a communication I/F (communication I/F 97 in FIG. 9), or may be display to a display unit (display unit 96 in FIG. 9).

### Procedure for Creating Relationship Graph

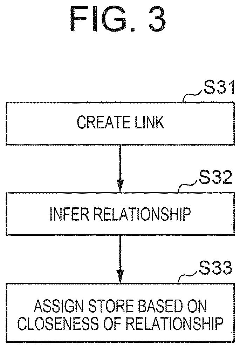

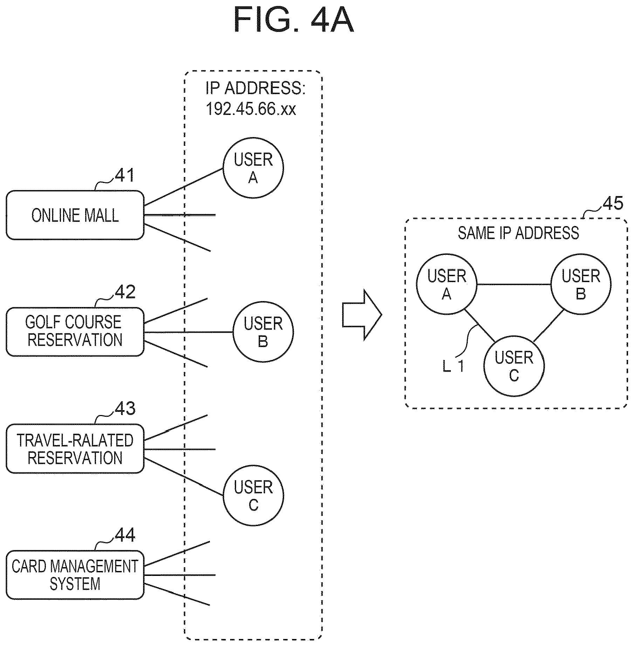

Next, a procedure for creating a relationship graph according to this embodiment will be described. Note that users A to E in the following description are users referred to for the description, and can be users of the user apparatuses 11. Also, the relationship graph is constituted by connections of user nodes circled in FIGS. 4A to 4D, and in the following description, the user nodes are simply referred to as users. FIG. 3 shows a flowchart of processing for creating the relationship graph, which is executed by the graph creation unit 102 according to the present embodiment. Each step of the processing in FIG. 3 will be described below.

### Step S31: Link Creation

In step S31, the graph creation unit 102 predicts and creates links between a plurality of users.

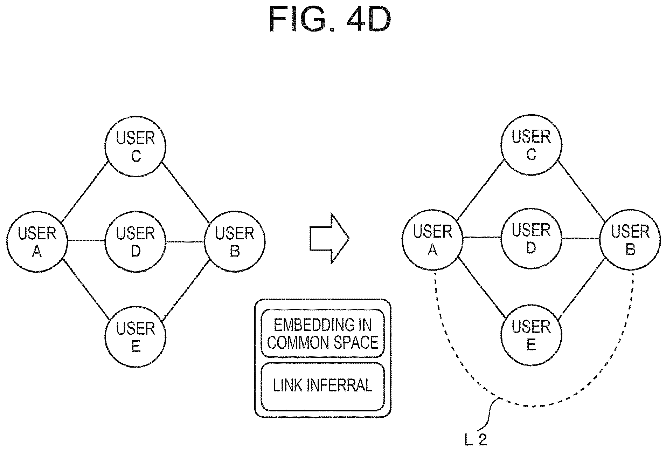

The processing for creating links will be described with reference to FIGS. 4A to 4D. FIGS. 4A to 4C are diagrams for illustrating an explicit link, and FIG. 4D is a diagram for illustrating an implicit link. An explicit link is a link created by explicit features held in common by two users (a user pair). An implicit link is a link created as an indirect relationship using explicit links that have already been created, although there is no clear explicit feature held in common by the user pair. Thus, links between users are identified by explicit links and implicit links.

FIG. 4A shows an example in which explicit links are created using an IP address of user apparatuses of users as a common feature. FIG. 4A shows an example in which an online mall 41, a golf course reservation service 42, a travel-related reservation service 43, and a card management system 44 exist as web services available to users A to C. In FIGS. 4A to 4C, four web services are shown, but the number of web services is not limited to a specific number.

The online mall 41 is a shopping mall that is available online (using the Internet). For example, the online mall 41 can provide a wide variety of products and services such as fashion, books, food, concert tickets, and real estate.

The golf course reservation service 42 is operated by a website that provides a service relating to a golf course online, and for example, can provide a search for golf courses, reservations, and lesson information.

The travel-related reservation service 43 is operated by a website that provides various travel services that are available online. The travel-related reservation service 43 can, for example, provide reservations for hotels and travel tours, reservations for airline tickets and rental cars, sightseeing information, hotels, and information on surrounding areas of hotels.

The card management system 44 is operated by a website that provides a service related to a credit card issued and managed by a predetermined card management company. The card management system 44 may also provide a service relating to at least one of the online mall 41, the golf course reservation service 42, and the travel-related reservation service 43.

In the example of FIG. 4A, the users A to C use the same IP address (=198.45.66.xx) to use the online mall 41, the golf course reservation service 42, and the travel-related reservation service 43. The information on the IP address can be acquired by the user feature acquisition unit 101.

In such a case, the graph creation unit 102 creates explicit links between the users A to C (e.g., a link L1 between the user A and the user C) with the feature of having the same IP address, as shown in a link state 45.

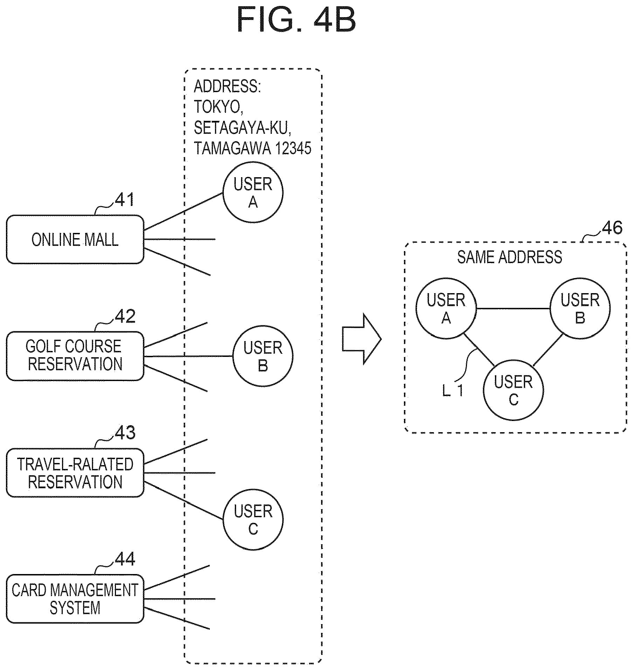

FIG. 4B shows an example of creating explicit links using a feature of an address of users as a common feature. Similarly to FIG. 4A, FIG. 4B shows an example in which an online mall 41, a golf course reservation service 42, a travel-related reservation service 43, and a card management system 44 exist as web services that are available to the users A to C. Here, the users A to C respectively use the online mall 41, the golf course reservation service 42, and the travel-related reservation service 43 by registering the same address (delivery address). Information on the address can be acquired by the user feature acquisition unit 101.

In such a case, the graph creation unit 102 creates explicit links between the users A to C (for example, a link L1 between the user A and the user C) with the feature of having the same address, as shown in a link state 46.

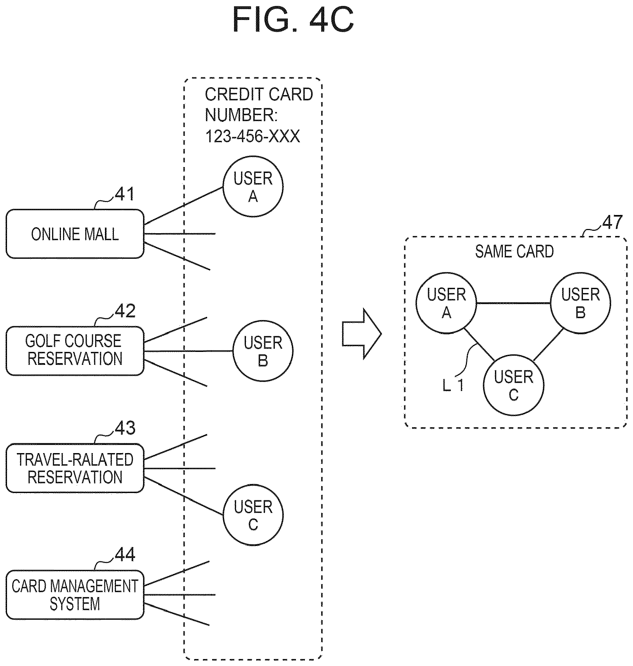

FIG. 4C shows an example of creating explicit links using a feature of credit card numbers used by users as a common feature. Similarly to FIG. 4A, FIG. 4C shows an example in which an online mall 41, a golf course reservation service 42, a travel-related reservation service 43, and a card management system 44 exist as web services that are available to the users A to C. Here, the users A to C each register the same credit card to use the online mall 41, the golf course reservation service 42, and the travel-related reservation service 43. Information including the credit card number can be acquired by the user feature acquisition unit 101.

In such a case, the graph creation unit 102 creates explicit links between the users A to C (for example, a link L1 between the user A and the user C) with the feature of having the same card, as shown in a link state 47.

FIG. 4D shows an example of creating an implicit link between users. In the example of FIG. 4D, the user C, the user D, and the user E are connected to the user A by explicit links, and the user C, the user D, and the user E are connected to the user B by explicit links. This kind of link feature (a feature indicating a relationship between links) is embedded in the common feature space, and a link obtained by inferring that a relationship is implicitly constructed between users (nodes) is created (established) as an implicit link. In the example of FIG. 4D, the user A and the user B are not connected by an explicit link, but an implicit link L2 is created as a result of inferring that there is a relationship in the common feature space. Note that the graph creation unit 102 predicts and creates explicit links between users by performing learning (representation learning, relationship learning, embedding learning, knowledge graph embedding) of a user relationship graph constituted by nodes (users) connected by explicit links. At this time, the graph creation unit 102 may perform the learning based on a known embedding model or its extension, as appropriate.

### Step S32: Inferring Relationships Between Links

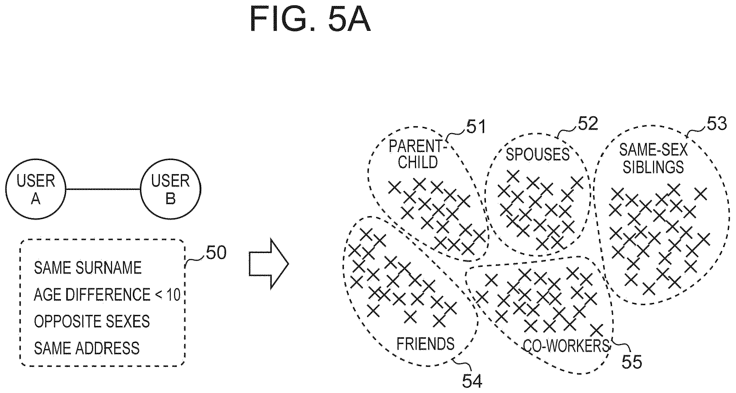

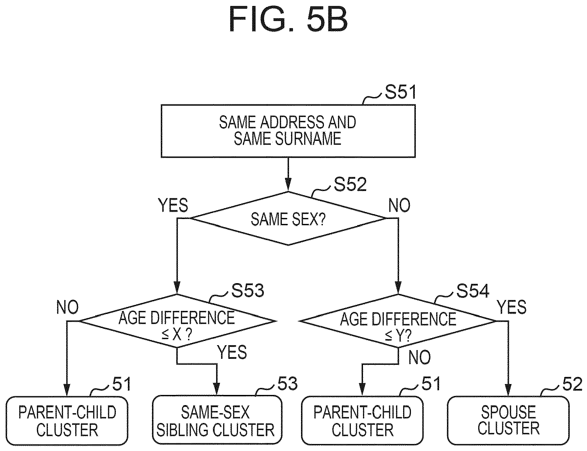

In step S32, the graph creation unit 102 infers relationships between the links predicted and created in step S31. The processing for inferring relationships between links will be described with reference to FIGS. 5A and 5B. FIG. 5A is a diagram for illustrating processing for inferring relationships between links, and shows an example of inferring a relationship of a link between the user A and the user B who are connected by an explicit link.

The graph creation unit 102 treats the pair of users connected by the link created in step S31 as a data point and groups the pair (the data point) into a cluster representing a common type, using various types of information acquired by the user feature acquisition unit 101. The various types of information can be information such as an IP address, an address, a credit card, an age, a sex, or a friend. Also, each cluster can be a cluster having a relationship such as spouses, a parent and child, neighbors, people sharing the same household, co-workers, friends, siblings of the same sex, or siblings of different sexes. In the example of FIG. 5A, a pair of users is indicated by an X mark, and a parent-child cluster 51, a spouse cluster 52, a same-sex sibling cluster 53, a friend cluster 54, and a co-worker cluster 55 are shown as clusters into which the pair can be grouped. Note that although five clusters are shown in FIG. 5A, the number of clusters is not limited to a specific number.

For example, if the user A and the user B have (share) features 50 of having the same surname, having an age difference of 10 years or less, being of opposite sexes, and having the same address, the graph creation unit 102 can group the pair of the user A and the user B into the cluster (spouse cluster 52) indicating the relationship of husband and wife (spouses).

FIG. 5B shows a flowchart of an example of processing for grouping pairs into clusters, which is executed by the graph creation unit 102.

At the start of step S51, it is assumed that the pair to be grouped has the features of having the same address and the same surname. In step S52, the graph creation unit 102 determines whether or not the target pair has the feature of being of the same sex. If the target pair has the feature of being of the same sex (Yes in step S52), the graph creation unit 102 determines in step S53 whether or not the age difference of the target pair is a predetermined threshold value (=X value) or less. If the age difference of the target pair is greater than the X value (No in step S53), the graph creation unit 102 groups the target pair into the parent-child cluster 51. If the age difference is the X value or less (Yes in step S53), the graph creation unit 102 groups the target pair into the same-sex siblings cluster 53. If the target pair does not have the feature of being of the same sex (No in step S52), the graph creation unit 102 determines in step S54 whether or not the age difference of the target pair is a predetermined threshold value (=Y value) or less. If the age difference is greater than the Y value (No in step S54), the graph creation unit 102 groups the target pair into the parent-child cluster 51. If the age difference is the Y value or less (Yes in step S54), the graph creation unit 102 groups the target pair into the spouse cluster 52.

### Step S33: Score Assignment Based on Closeness of Relationship

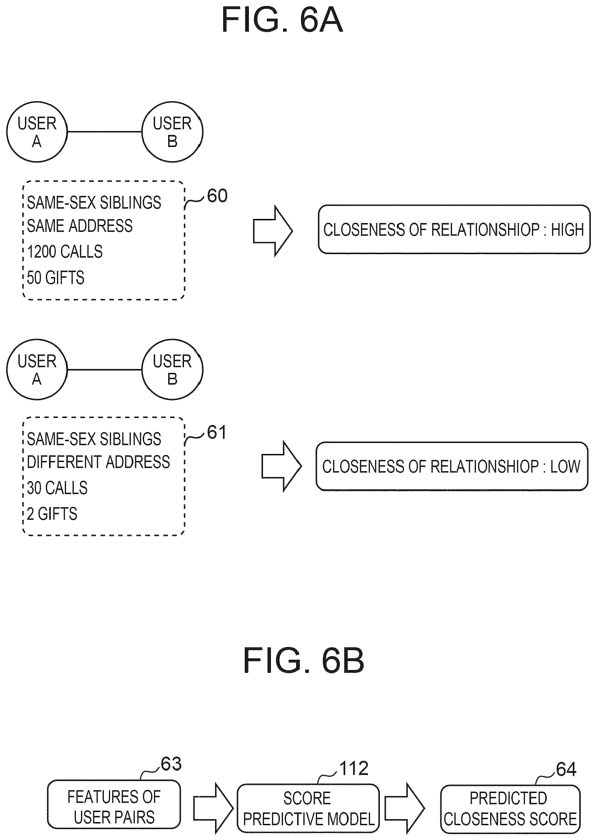

In step S33, the graph creation unit 102 predicts a score based on the closeness of the relationship for the pair inferred in step S32, and assigns the score to the pair. In this embodiment, the score is a numeric value between 0 and 1, but there is no particular limitation on the numeric value that the score can take. FIG. 6A shows a conceptual diagram of a score based on the closeness of the relationship for a user pair (hereinafter referred to as a closeness score).

In the example of FIG. 6A, the closeness of the relationship between the pair of users changes depending on the features that the user A and the user B connected by the explicit link have (share). In the upper part of FIG. 6A, if the user A and the user B have the features 60 of being same-sex siblings, having the same address, having a call history of 1200 calls, and exchanging gifts 50 times, the closeness of the relationship between the pair of users (i.e., the closeness score) is higher. On the other hand, in the lower part of FIG. 6A, if the user A and the user B have features 61 of being same-sex siblings, having different addresses, having a call history of 30 calls, and exchanging two gifts, the closeness of the relationship between the pair of users (i.e., the closeness score) is lower. In this manner, as in the example of FIG. 6A, even if the user A and the user B are same-sex siblings, the closeness of the relationship between the pair differs depending on other features shared by the pair of users. It is observed that pairs with high relationship closeness have a close social distance to each other and have a high influence on each other. On the other hand, it is observed that pairs with low relationship closeness have a far social distance from each other and do not have a close relationship.

In this embodiment, a score prediction model 112 is used to predict the closeness score for a user pair. Schematic architecture of the score prediction model 112 is shown in FIG. 6B. The score prediction model 112 is a learning model that receives features 63 of the user pair as input and predicts the closeness score 64 for the features 63.

The score prediction model 112 is, for example, a learning model that performs weak supervised learning, such as a learning model using a convolutional neural network (CNN). In the present embodiment, the score prediction model 112 is a learning model that is trained using closeness scores (0 to 1) attached to a plurality of features for user pairs as training data, as shown in FIG. 6A. For example, in the training stage, combined data of a closeness score close to 1 set for the features 60 in FIG. 6A and a closeness score close to 0 set for the features 61 is used as training data. The training processing is performed by the training unit 105. It should be noted that the score prediction model 112 may be different for each type of relationship of a user pair, and may be a learning model trained according to one type of relationship.

It should be noted that, in the present embodiment, although the closeness score for a user pair is predicted using the score prediction model 112, the graph creation unit 102 may also be configured to predict the score using another method.

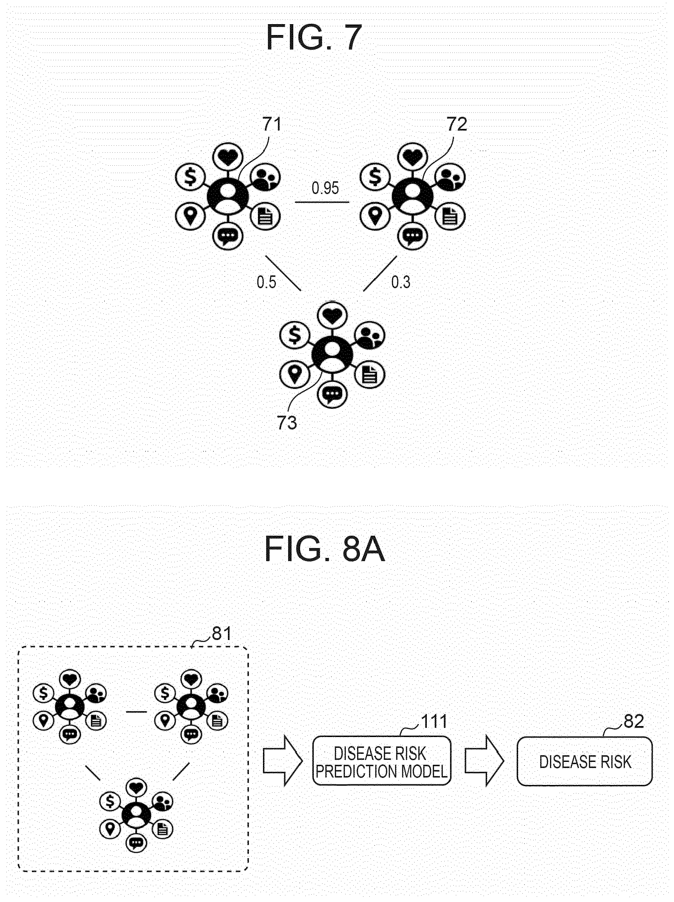

Through the above processing, explicit links or implicit links are formed between a plurality of users, closeness scores are assigned for each link, and a relationship graph is created. FIG. 7 shows a conceptual diagram of a relationship graph. Each user 71 to 73 has a plurality of features, and pairs of users are assigned predicted closeness scores as described above.

### Disease Risk Prediction Processing

Next, disease risk prediction processing according to the present embodiment will be described. In this embodiment, the prediction unit 104 uses the trained disease risk prediction model 111 to predict the disease risk for the target user. FIG. 8A shows a diagram for illustrating the training stage of the disease risk prediction model 111. The disease risk prediction model 111 is a learning model that receives the features 81 based on the relationship graph created by the graph creation unit 102 as input, and predicts and outputs the risk of developing (contracting) at least one predetermined disease (disease risk 82) for each user. The disease risk prediction model 111 is, for example, a learning model based on a convolutional neural network (CNN).

In the training stage, the training unit 105 first prepares the features 81, which are input data to the disease risk prediction model 111, based on the relationship graph created by graph creation unit 102.

Based on the relationship graph, the training unit 105 identifies a pair composed of any user and a predetermined disease (e.g., diabetes) that the user has actually had (or currently has; the same applies hereinafter), and assigns a positive flag to the pair. If the user has had multiple diseases, a positive flag is assigned to each pair composed of the user and the disease. The presence or absence of the predetermined disease (presence or absence of contraction of the disease) can be determined from the medical history and the like of each user included in the user features 121. Furthermore, based on the relationship graph, the training unit 105 identifies a pair composed of a user who does not have the predetermined disease and information set as appropriate, and assigns a negative flag to the pair. The training unit 105 defines a pair with a positive flag as a positive data point and a pair with a negative flag as a negative data point. Note that the positive flag and the negative flag may be any value or information as long as they can be distinguished from each other. The training unit 105 can acquire disease features for each of a plurality of users from the relationship graph by assigning such flags. The disease features include information indicating the presence or absence of one or more predetermined diseases.

Subsequently, the training unit 105 defines individual features of each user (e.g., demographic information, weight, medical history) and features of user pairs (e.g., the above-mentioned closeness score, or at least one feature shared between pairs) as the input data (features 81) of the disease risk prediction model 111. It should be noted that the disease features described above are given to (or included in) the personal features of each user or the features of the user pairs.

The training unit 105 uses the input data (feature 81) prepared in this manner to train the disease risk prediction model 111 so as to output the risk of developing (contracting) at least one predetermined disease (disease risk 82) for each user. In this embodiment, disease risk is represented by the likelihood of developing each disease (e.g., a numerical value from 0 to 1, where 1 indicates the maximum likelihood). Since each user's medical history changes over time, the training unit 105 can train the disease risk prediction model 111 each time the graph creation unit 102 updates the relationship graph.

In the prediction stage, the prediction unit 104 predicts the disease risk for the target user. FIG. 8B shows a diagram for illustrating the disease risk prediction stage for the target user. The prediction unit 104 inputs the user features 83 of the target user set by the target user feature setting unit 103 to the trained disease risk prediction model 111, and predicts a disease risk 84 for the target user. The disease risk 84 indicates the risk of developing at least one predetermined disease. The user features 83 may be any information by which the target user can be identified in the relationship graph among the user features 121 stored in the user feature storage unit 120, and for example, can be the address, name, demographic information, or the like of the target user.

As described above, in this embodiment, the disease risk is represented by a numeric value between 0 and 1 for each disease. When the disease risk 84 for a certain disease, which is output from the disease risk prediction model 111, is higher than a predetermined threshold, the prediction unit 104 can determine that the risk of developing the disease is high. The predetermined threshold is, for example, 0.7.

In this manner, the prediction unit 104 predicts the disease risk of the target user based on the disease risk prediction model 111 trained based on the relationship graph. As a result, for example, it is possible to accurately predict the risk of developing a genetic disease caused by blood ties (parents and children, etc.). Furthermore, it is possible to accurately predict the risk of developing infectious diseases that can be transmitted between people who live in physically close environments, such as family members who live together and co-workers who work in the same company.

### Hardware Configuration of Information Processing Apparatus 10

FIG. 9 is a block diagram showing an example of a hardware configuration of the information processing apparatus 10 according to this embodiment.

The information processing apparatus 10 according to the present embodiment can be implemented also on any one or more computers, mobile apparatuses, or other processing platforms.

With reference to FIG. 9, an example is shown in which the information processing apparatus 10 is implemented on a single computer, but the information processing apparatus 10 according to the present embodiment may be implemented on a computer system including a plurality of computers. The plurality of computers may be connected so as to be capable of mutual communication through a wired or wireless network.

As shown in FIG. 9, the information processing apparatus 10 may include a CPU 91, a ROM 92, a RAM 93, an HDD 94, an input unit 95, a display unit 96, a communication I/F 97, and a system bus 98. The information processing apparatus 10 may include an external memory.

The CPU (Central Processing Unit) 91 performs overall control of operations in the information processing apparatus 10, and controls each constituent unit (92 to 97) via the system bus 98, which is a data transmission path.

The ROM (Read Only Memory) 92 is a non-volatile memory that stores control programs and the like needed for the CPU 91 to execute processing. Note that the program may also be stored in a non-volatile memory such as the HDD (Hard Disk Drive) 94 or an SSD (Solid State Drive), or an external memory such as a detachable storage medium (not shown).

The RAM (Random Access Memory) 93 is a volatile memory and functions as a main memory, a work area, and the like of the CPU 91. That is, during execution of processing, the CPU 91 executes various functional operations by loading necessary programs and the like from the ROM 92 to the RAM 93, and executing the programs and the like. The learning model storage unit 110 and the user feature storage unit 120 shown in FIG. 2 can be constituted by the RAM 93.

The HDD 94 stores various types of data, various types of information, and the like that are needed when the CPU 91 performs processing using a program. Also, the HDD 94 stores various types of data, various types of information, and the like obtained by the CPU 91 performing processing using a program or the like.

The input unit 95 is constituted by a keyboard or a pointing apparatus such as a mouse.

The display unit 96 is constituted by a monitor such as a liquid crystal display (LCD). The display unit 86 may also function as a GUI (Graphical User Interface) due to being included in combination with the input unit 95.

The communication I/F 97 is an interface that controls communication between the information processing apparatus 10 and an external apparatus.

The communication I/F 97 provides an interface with a network and executes communication with an external apparatus via the network. Various types of data, various types of parameters, and the like are transmitted and received to and from the external apparatus via the communication I/F 97. In this embodiment, the communication I/F 97 may execute communication via a wired LAN (Local Area Network) or a dedicated line conforming to a communication standard such as Ethernet (registered trademark). However, the network that can be used in this embodiment is not limited thereto, and may also be constituted by a wireless network. This wireless network includes a wireless PAN (Personal Area Network) such as Bluetooth (registered trademark), ZigBee (registered trademark), and UWB (Ultra Wide Band). This wireless network also includes a wireless LAN (Local Area Network) such as Wi-Fi (Wireless Fidelity) (registered trademark) and a wireless MAN (Metropolitan Area Network) such as WiMAX (registered trademark). Furthermore, the wireless network includes a wireless WAN (Wide Area Network) such as LTE/3G, 4G, and 5G. Note that it is sufficient that the network connects the apparatuses such that communication is possible therebetween and is capable of communication, and the standard, scale, and configuration of communication is not limited to the above.

The function of at least some of the elements of the information processing apparatus 10 shown in FIG. 2 can be realized by the CPU 91 executing a program. However, the function of at least some of the elements of the information processing apparatus 10 shown in FIG. 2 may also be realized by an operation of dedicated hardware. In this case, the dedicated hardware operates based on control performed by the CPU 91.

### Hardware Configuration of User Apparatus 11

The hardware configuration of the user apparatus 11 shown in FIG. 1 may be the same as that shown in FIG. 9. That is, the user apparatus 11 can include the CPU 91, the ROM 92, the RAM 93, the HDD 94, the input unit 95, the display unit 96, the communication I/F 97, and the system bus 98. The user apparatus 11 can display various types of information provided by the information processing apparatus 10 on the display unit 96 and perform processing corresponding to an input operation received from the user via the GUI (constituted by the input unit 95 and the display unit 96).

### Flow of Processing

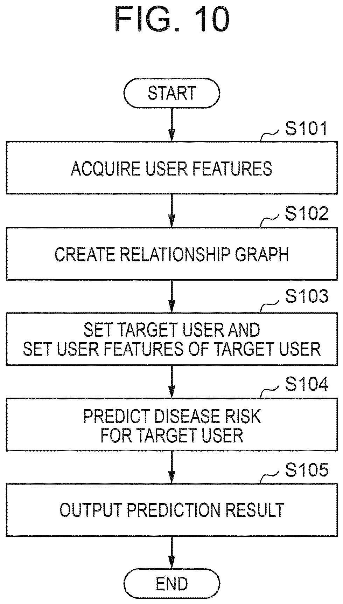

FIG. 10 shows a flowchart of processing executed by the information processing apparatus 10 according to this embodiment. The processing shown in FIG. 9 can be realized by the CPU 91 of the information processing apparatus 10 loading a program stored in the ROM 92 or the like to the RAM 93 and executing the loaded program. The information processing system shown in FIG. 1 will be referred to for the description of FIG. 10. The attribute prediction model 111 and the score prediction model 112 trained by the training unit 105 are stored in the learning model storage unit 110.

In step S101, the user feature acquisition unit 101 acquires the user features of the users from the user apparatuses 11-1 to 11-N and stores the acquired user features in the user feature storage unit 120 as the user features 121. The processing of step S101 may also be processing for acquiring (collecting) user features of a predetermined past period.

In step S102, the graph creation unit 102 uses the various user features acquired by the user feature acquisition unit 101 to create a relationship graph for the users 1 to N. The procedure for creating the relationship graph is as described above.

In step S103, the target user feature setting unit 103 sets a user (target user) whose attribute is to be predicted from among the users 1 to N. As described above, the target user may be set by the operator through an input operation performed using the input unit 95, may be set in the system in advance, or may be set by any program stored in the ROM 92 or the RAM 93. Furthermore, in step S103, the target user feature setting unit 103 acquires the user features of the target user from the user features 121 and sets the acquired user features in the prediction unit 104.

In step S104, the prediction unit 104 inputs the user features of the target user set in step S103 to the disease risk prediction model 111, and predicts the disease risk, which indicates the risk of developing (contracting) at least one predetermined disease for the target user.

In step S105, the output unit 106 outputs the disease risk prediction result for the target user predicted in step S104. The output unit 106 may generate information about the prediction result and output the generated information to an external apparatus (not shown).

For example, as in this embodiment, the disease risk is represented by a numeric value from 0 to 1, a first threshold is set to 0.1, a second threshold is set to 0.3, a third threshold is set to 0.7, and the output unit 106 may generate and output low-level to high-level information according to the thresholds. For example, if the disease risk of a first disease is 0.2, the disease risk is between the first threshold and the second threshold, and although it is low, it is not 0. Therefore, the output unit 106 may generate and output information indicating a warning regarding the first disease. Also, when the disease risk of a second disease is 0.5, the disease risk is between the second threshold and the third threshold, and the disease risk of the second disease is slightly high. Therefore, the output unit 106 may generate and output information indicating the likelihood of developing the second disease. Also, when the disease risk of a third disease is 0.8, the disease risk is the third threshold or higher, and thus the disease risk of the third disease is considerably high. Therefore, the output unit 106 may generate and output information indicating that the risk of developing the third disease is high.

In this manner, the information processing apparatus 10 creates a relationship graph network (relationship graph) indicating social relationships between users from the user features of a plurality of users, and based on the relationship graph, predicts disease risk for a target user. It is possible to assist the prediction of the possible risk of developing a disease for a target user (any user), in a more multifaceted manner.

It should be noted that although a specific embodiment has been described above, the embodiment is merely an example, and is not intended to limit the scope of the present invention. The apparatuses and methods described in the present specification can be embodied in forms other than those described above. Also, the above-described embodiment can be subjected to omission, replacement, and modification as appropriate without departing from the scope of the present invention. Modes obtained through such omission, replacement, and modification are encompassed in the description of the claims and the range of equivalency thereto, and belong to the technical scope of the present invention.

## Claims

1. An information processing apparatus, comprising:
acquisition means for acquiring factual features of each of a plurality of users as user features;
creation means for creating a relationship graph indicating a social relationship between the plurality of users based on the user features;
target user setting means for setting a target user among the plurality of users; and
prediction means for, based on the relationship graph and the user feature of the target user, predicting a disease risk indicating a risk of developing at least one disease for the target user.

2. The information processing apparatus according to claim 1, wherein the prediction means predicts the disease risk for the target user by using a machine learning model configured to receive the user feature of the target user as input and output the disease risk for the target user.

3. The information processing apparatus according to claim 2, further comprising
training means for training the machine learning model,
wherein the training means trains the machine learning model using a disease feature obtained from the relationship graph.

4. The information processing apparatus according to claim 3, wherein the disease feature obtained from the relationship graph includes information indicating whether or not each of the plurality of users has contracted the at least one predetermined disease.

5. The information processing apparatus according to any one of claims 1 to 4, wherein in the relationship graph, each user is indicated by a user node, and the creation means connects nodes with links based on the factual features.

6. The information processing apparatus according to claim 5, wherein the creation means connects a pair of user nodes having the same factual feature with an explicit link, and connects, with an implicit link, a pair of nodes that are not linked with the explicit link, based on a plurality of pairs of user nodes that are connected with the explicit links.

7. The information processing apparatus according to claim 5 or 6, wherein the creation means determines a closeness of the connected pair based on at least one factual feature shared by the pair.

8. The information processing apparatus according to any one of claims 1 to 7, wherein the disease risk is represented by a numeric value from 0 to 1 for each of the at least one disease, where 1 is a maximum likelihood.

9. An information processing method, comprising:
acquiring factual features of each of a plurality of users as user features;
creating a relationship graph indicating a social relationship between the plurality of users based on the user features;
setting a target user among the plurality of users; and
predicting, based on the relationship graph and the user feature of the target user, a disease risk indicating a risk of developing at least one disease for the target user.

10. An information processing program for causing a computer to execute information processing, the program causing the computer to execute:
acquisition processing for acquiring factual features of each of a plurality of users as user features;
creation processing for creating a relationship graph indicating a social relationship between the plurality of users based on the user features;
target user setting processing for setting a target user among the plurality of users; and
prediction processing for predicting, based on the relationship graph and the user feature of the target user, a disease risk indicating a risk of developing at least one disease for the target user.
